(19) Europäisches Patentamt
European Patent Office
Office européen des brevets

(11) **EP 1 348 457 B1**

(12) **EUROPÄISCHE PATENTSCHRIFT**

(45) Veröffentlichungstag und Bekanntmachung des Hinweises auf die Patenterteilung:
**25.05.2005   Patentblatt 2005/21**

(51) Int Cl.$^7$: **A61M 1/16**

(21) Anmeldenummer: **03002947.4**

(22) Anmeldetag: **11.02.2003**

(54) **Verfahren zur Bestimmung eines Behandlungsparameters an einer Hämofiltrationsvorrichtung und Hämofiltrationsvorrichtung zur Anwendung des Verfahrens**

Method of determining of a treatment parameter on a hemofiltration apparatus and hemodialysis apparatus therefor

Méthode pour déterminer des paramètres de traitement utilisant un appareil d'hémofiltration et dispositif pour cela

(84) Benannte Vertragsstaaten:
**AT BE BG CH CY CZ DE DK EE ES FI FR GB GR HU IE IT LI LU MC NL PT SE SI SK TR**

(30) Priorität: **19.03.2002  DE 10212247**

(43) Veröffentlichungstag der Anmeldung:
**01.10.2003   Patentblatt 2003/40**

(73) Patentinhaber: **Fresenius Medical Care Deutschland GmbH**
**61352 Bad Homburg v.d.H. (DE)**

(72) Erfinder:
• **Gross, Malte, Dr.**
  **97424 Schweinfurt (DE)**
• **Maierhofer, Andreas, Dr.**
  **97422 Schweinfurt (DE)**

(74) Vertreter: **Dreyhsig, Jörg, Dr.**
**Fresenius Medical Care AG,**
**Patent Department,**
**Frankfurter Strasse 6-8**
**66606 St. Wendel (DE)**

(56) Entgegenhaltungen:
**EP-A- 0 930 080          EP-A- 1 062 960**
**DE-A- 19 831 385**

**Beschreibung**

Die Erfindung betrifft das Gebiet von Verfahren zur Bestimmung von Eshandlungsparametern an einer Hämofiltrationsvorrichtung nach dem Oberbegriff des Anspruch 1 sowie Hämofiltrationsvorrichtungen nach dem Oberbegriff des Anspruch 10.

In der Nierenersatzbehandlung werden verschiedene Verfahren eingesetzt. Bei einigen dieser Verfahren wird einem Patienten während der Behandlung kontinuierlich Blut entnommen und in einen extrakorporalen Kreislauf eingespeist. Dort durchfließt es ein Blutreinigungselement, um danach zum Patient zurückgeführt zu werden. Das Blutreinigungselement weist meist einen durch eine semipermeable Membran in zwei Kammern geteiltes Filterelement auf, dessen eine Kammer vom Blut durchflossen wird. Gegenwärtig werden hierfür vor allem Filterelemente verwendet, die viele Tausend von Hohlfasern beinhalten, deren Inneres vom Blut durchflossen wird.

Bei der Hämodialyse wird die andere Kammer von einer Reinigungsflüssigkeit (Dialysierflüssigkeit) durchflossen, die aus dem Blut zu entfernende Stoffe wie z.B. Harnstoff per Diffusion aufnimmt und bzgl. anderer, im Blut zu belassender Stoffe wie Elektrolyte eine Zusammensetzung ähnlich eines gesunden Blutbildes aufweist. Auszuscheidene Flüssigkeitsvolumina werden mittels einer Komponente, die die Ultrafiltration steuert, ebenfalls von der Blutkammer zur Dialysierflüssigkeitskammer des Filterelementes entfernt.

Bei der Hämofiltration wird die andere Kammer des Filterelementes, die fortan als erste Kammer bezeichnet wird, nicht durch eine zweite Flüssigkeit vollständig durchflossen. Vielmehr wird in diese Kammer nur Ultrafiltrat über die Membran hinzugeführt, das dann über eine Ultrafiltratableitung abgeführt wird. Dabei wird die entfernte Flüssigkeitsmenge weit über der gehalten, die dem Patienten zur Erreichung seines Trockengewichtes entfernt werden muß. Auf diese Weise werden zu entfernende Stoffe wie Harnstoff in nennenswertem Umfang durch Konvektion mit dem Ultrafiltrat abgeführt. Gleichzeitig wird fast die gesamte Flüssigkeitsmenge durch eine Substitutionsflüssigkeit ersetzt, die dem Patienten an geeigneter Stelle über den extrakorporalen Kreislauf zurückgegeben wird.

Da Konvektion und Diffusion verschiedengroße Moleküle unterschiedlich effektiv durch die Membran entfernen können, wird auch die Kombination beider Verfahren - eine Hämodiafiltrationsbehandlung - angewendet. Moderne Dialysemaschinen weisen hierzu die Möglichkeit auf, zwischen diesen Behandlungsmodi zu wechseln, ohne das es eines komplexen Umbaus bedarf. Dabei weisen einige bekannte Geräte die Möglichkeit auf, die Dialysier- und die Substitutionsflüssigkeit online während der Behandlung aus Wasser und entsprechenden Konzentrat durch die Maschine bereitzustellen. Bei diesen Vorrichtungen ist es nicht mehr notwendig, enorme Flüssigkeitsmengen dieser Flüssigkeiten (bis ca. 200 Liter) in Form von Beuteln bereitzuhalten. Eine solche Vorrichtung ist z.B. Gegenstand der EP 0 930 080 A1.

Um den Erfolg einer Nierenersatzbehandlung überwachen zu können, ist die Bestimmung von Behandlungsparameters an solchen Blutreinigungsgeräten, insbesondere der Leistungsfähigkeit des Blutreinigungselementes, von großem Interesse. Als Leistungsfähigkeit wird zumeist die Clearance des Blutreinigungselementes angegeben.

Die Clearance K ist als der Blutstrom definiert, der durch das Blutreinigungselement vollständig von einer Substanz (z.B. Harnstoff) befreit wird. Dabei wird bei einer Hämodialysebehandlung vorausgesetzt, daß die Dialysierflüssigkeit beim Eintritt in den Dialysator die zu entfernende Substanz nicht enthält. Die Clearance ist von Fläche und Material des Dialysators und den jeweiligen Betriebsbedingungen (Blut-, Dialysierflüssigkeits- und Ultrafiltrationsfluß) abhängig. Die Clearance kommt sowohl durch Diffusion als auch durch Konvektion über die Membran des Filterelementes - des Dialysators - zustande.

Der Begriff der Clearance läßt sich auch auf Substanzen wie z.B. Natrium-Ionen erweitern, die bereits in der Dialysierflüssigkeit vorhanden sind. In diesem Fall spricht man von der Dialysance D. Sie ist als der Blutfluß definiert, der vollständig auf das Konzentrationsniveau in der Dialysierflüssigkeit gebracht wird.

Aus der Clearance K kann die dimensionslose Größe Kt/V berechnet werden, wobei t die Behandlungsdauer und V das Verteilungsvolumen der Substanz im menschlichen Körper ist. Kt/V für Harnstoff wird weitverbreitet als Maß für die Effizienz einer Dialysebehandlung angewendet.

Die Messung der Harnstoffkonzentration ist jedoch bisher relativ aufwendig. Entweder erfordert sie die Entnahme von Blutproben, was für den Patienten mit Unannehmlichkeiten einhergeht und zudem keine schnelle, automatisierte Auswertung ermöglicht, oder sie gestaltet sich als Messung in der verbrauchten Dialysierflüssigkeit immer noch recht aufwendig.

Eine Alternative besteht gegenwärtig in der Bestimmung der ionischen Dialysance. Das Grundprinzip dieser Messungen beruht auf der Tatsache, daß Harnstoff und kleine Ionen wie $Na^+$, $Cl^-$, etc. ein nahezu identisches Diffusionsverhalten haben. Die Konzentration dieser Ionen läßt in der Dialysierflüssigkeit leicht mit Hilfe von Messungen der elektrischen Leitfähigkeit bestimmen, welche mit relativ einfach aufgebauten Meßzellen ermittelt werden kann. Anstelle der Harnstoff-Clearance wird daher zunächst die Ionen-Dialysance bestimmt. Diese kann dann - aufgrund des gleichen zu erwartenden Diffusionsverhaltens - gleich der Harnstoff-Clearance gesetzt werden.

Im Stand der Technik finden sich diverse Veröffentlichungen zur Berechnung der Dialysance (z.B. J. Sargent und F. Gotch, in: Replacement of Renal Functions by Dialysis, ed. C. Jacobs et al., Kluwer, Dordrecht, Boston, London, 1996, S. 39). Ohne Ultrafiltration läßt sie sich in der sogenannten diälysatseitigen Form in der folgenden Form ausdrücken:

$$D = Qd \ \frac{Cdo - Cdi}{\alpha Cbi - Cdi}$$ (1),

wobei

Qd: Dialysierflüssigkeitsfluß,

Cdo: Konzentration des betrachteten Stoffes in der abgeführten Dialysierflüssigkeit,

Cdi: Konzentration des betrachteten Stoffes in der zugeführten Dialysierflüssigkeit,

Cbi: Konzentration des betrachteten Stoffes im in den extrakorporalen Kreislauf einströmenden Blut (wobei nur der Volumenanteil zu betrachten ist, in dem dieser Stoff effektiv gelöst ist),

$\alpha$: Gibbs-Donnan-Faktor.

Der Gibbs-Donnan Faktor berücksichtigt, daß auf der Blutseite geladene Ionen wie $Na^+$ teilweise an entgegengesetzt geladene, nicht dialysatorgängige Proteine gebunden werden. Dieser Effekt hat zur Folge, daß sich im diffusiven Gleichgewicht (bei verschwindenden Flüssen) im Blutplasma eine etwas höhere Ionen-Konzentration als in der Dialysierflüssigkeit einstellen würde, da ein elektrisches Feld der Diffusion entgegenwirkt. Für den in der Praxis besonders relevanten Fall der Natrium-Ionen im Blutplasma liegt $\alpha$ bei ca. 0,95. Sollte es die Genauigkeit nicht erfordern, kann dieser Faktor auch vernachlässigt werden.

In der Gleichung 1 können alle Größen außer Cbi leicht gemessen werden. Dazu genügt es, zwei Leitfähigkeitsmeßzellen im Dialysierflüssigkeitskreislauf anzuordnen, die jeweils die Leitfähigkeiten am Eingang und Ausgang des Dialysators bestimmen. Letztere lassen sich leicht in die Konzentrationen Cdi und Cdo umrechnen. Falls die Konzentration Cdi auch vorgegeben und daher bekannt ist, weil z.B. genau definierte Flüssigkeiten verwendet werden, kann sich die Messung von Cdi sogar erübrigen. Der Dialysierflüssigkeitsfluß Qd wird meist durch die Hämodialysemaschine vorgegeben und ist daher ebenfalls bekannt. Andernfalls können natürlich zusätzlich entsprechende Sensoren vorgesehen sein.

Aus praktischen Gründen sind Leitfähigkeitsmessungen auf der Blutseite aber problematisch. Es ist jedoch möglich, durch eine Änderung der Konzentration Cdi den Term Cbi zu eliminieren. Dies kann z.B. in Form einer Konzentrationsstufe oder eines Bolus geschehen. Ersteres ist in der DE 39 38 662 A1 beschrieben, letzteres in der DE 197 47 360 A1 oder WO 00/02604 A1 (auf diese Schriften wird hiermit explizit Bezug genommen). Beide Möglichkeiten sollen im folgenden als Alternativen für eine Änderung der Konzentration in einer frischen Flüssigkeit gelten, die für die Blutbehandlung benötigt wird. Die Dialysance kann dann folgendermaßen bestimmt werden:

$$D = Qd(1 - \frac{Cdo2 - Cdo1}{Cdi2 - Cdi1}) = Qd(1 - \frac{\Delta Cdo}{\Delta Cdi})$$ (2),

wobei

Cdi1,2: Cdi vor und nach (Stufe) bzw. außerhalb und während (Bolus) der Änderung

Cdo1,2: Cdo vor und nach (Stufe) bzw. außerhalb und während (Bolus) der Änderung.

Im Falle einer Stufenänderung stellen $\Delta$Cbi bzw. $\Delta$Cdo einfache Differenzen dar, im Falle der Bolusmethode wird darunter die über den Bolus integrierte Änderung relativ zu einem Basisniveau verstanden.

Mit Hilfe von D kann nun auch Cbi mit Gleichung 1 bestimmt werden. Dabei wäre als äquivalent anzusehen, zunächst Cbi als zu bestimmenden Parameter aus einer zu Gleichung 2 entsprechenden Gleichung zu bestimmen, die aus Gleichung 1 hervorgeht, wenn D eliminiert wird.

Es sind weitere Verfahren im Stand der Technik wie die WO 98/32476 A1 oder EP 0 658 352 A1 bekannt, die nicht explizit die Gleichung 2 zur Bestimmung von D einsetzen, aber letztendlich immer auf dem Prinzip beruhen, eine Änderung einer physikalischen-chemischen Eigenschaft Cdi herbeizuführen und die entsprechende Änderung Cdo festzuhalten, um zu einer Aussage über die physikalisch-chemische Eigenschaft Cbi auf der Blutseite oder die Filterleistungsfähigkeit D zu gestatten.

Im Stand der Technik werden dabei ausnahmslos Verfahren angegeben, die eine Bestimmung während einer Hämodialysebehandlung erlauben. Zwar finden sich dort auch - zum Teil unterschiedliche - Angaben, wie der während einer Hämodialysebehandlung dem Blut entzogene Ultrafiltrationfluß Qf in den Gleichungen (1) und (2) berücksichtigt werden kann. Beispielhaft sei hier die EP 1 062 960 A2 genannt, wonach Qd durch die Summe der Flüsse Qd und Qf ersetzt wird. Bei einer Hämodialysebehandlung ist jedoch der Ultrafiltrationsfluß Qf sehr klein im Vergleich zum Dialysierflüssigkeitsfluß Qd und auch zum Blutfluß Qb, d.h. es handelt sich um einen verhältnismäßig kleinen Störeffekt. So liegen z.B. typische Werte bei Qf = 15 ml/min, Qd = 500 ml/min und Qb = 300 ml/min.

Bei einer Nierenersatzbehandlung ist aber die Kenntnis der Leistungsfähigkeit des Blutreinigungselementes genauso von Interesse, wenn es sich um eine Hämofiltrationsbehandlung handelt - sei es allein oder in Kombination mit einer Hämodialysebehandlung in Form einer Hämodiafiltrationsbehandlung.

In diesem Zusammenhang ist zunächst nicht offensichtlich, wie das Konzept der Ionen-Dialysance auf diesen Fall übertragen werden kann. Bei der Hämofiltration gibt es zunächst gar keinen Dialysator, der von einer Dialysierflüssigkeit durchflossen wird. Bei Hämofiltration und -diafiltration werden zudem sehr große Flüssigkeitsmengen über einen Hämofilter oder -dialysator entzogen und gleichzeitig an anderer Stelle dem extrakorporalen Blutkreislauf wieder zugegeben. Dabei werden Flüsse bis zu 100 ml/min erreicht, da nicht mehr als sehr klein gegenüber dem Dialysierflüssigkeitsfluß (bei der Hämodiafiltration) und dem, Blutfluß angesehen werden können.

Der Erfindung liegt daher die Aufgabe zugrunde, ein Verfahren bereitzustellen, mit dem ein Behandlungsparameter auch an einer Hämofiltrationsbehandlung auf einfache Art und Weise bestimmt werden kann, wobei weiterhin auf blutseitige Messungen verzichtet werden soll. Der Erfindung liegt auch die Aufgabe zugrunde, eine entsprechende Hämofiltrationsvorrichtung zur Anwendung des erfindungsgemäßen Verfahrens bereitzustellen. Der Fall einer gleichzeitig zusätzlichen Hämodialysebehandlung (Hämodiafiltration) soll dabei explizit miteingeschlossen werden, d.h. bei der Hämofiltrationsbehandlung kann es sich auch um eine Hämodiafiltrationsbehandlung handeln.

Die Lösung gelingt durch die Merkmale des Anspruchs 1 bzw. 10. Vorteilhafte Ausführungsformen sind Gegenstand der Unteransprüche.

Die Erfindung sowie eine beispielhafte Ausführungsform einer erfindungsgemäßen Hämodiafiltrationsvorrichtung werden anschließend anhand der Zeichnungen näher erläutert. Es zeigen:

Fig. 1:  Eine schematisierte Darstellung der Flüssigkeitsparameter bei der Hämodialyse,

Fig. 2a:  eine schematisierte Darstellung der Flüssigkeitsparameter der Hämofiltration mit Prädilution,

Fig. 2b:  eine schematisierte Darstellung der Flüssigkeitsparameter der Hämofiltration mit Postdilution und

Fig. 3:  eine schematisierte Darstellung einer Ausführungsform einer erfindungsgemäßen Hämodiafiltrationsvorrichtung.

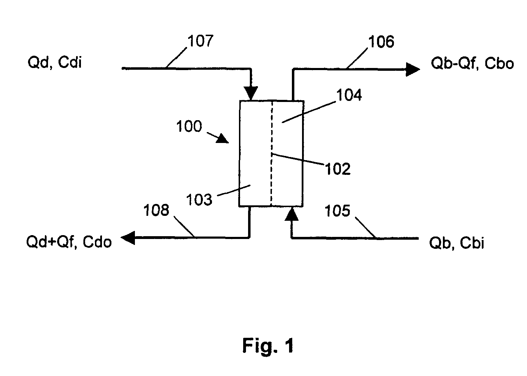

Die Erfindung basiert auf der überraschenden Erkenntnis, daß sich weite Teile des bei einer Hämodialyse angewendeten Formalismus auf die Hämofiltration übertragen lassen, wenn die richtige Zuordnung der beteiligten Parameter vorgenommen wird. Hierzu sei zunächst auf die Fig. 1 verwiesen: Bei einer Hämodialysebehandlung wird ein Hämodialysator 100, der durch eine semipermeable Membran 102 in zwei Kammern 103 und 104 geteilt, wobei in die erste Kammer 103 frische Dialysierflüssigkeit über eine Dialysierflüssigkeitszuführleitung 107 mit dem Fluß Qd und einer physikalisch-chemischen Eigenschaft Cdi fließt. Aus dieser Kammer 103 fließt über eine Dialysierflüssigkeitsabführleitung 108 ein um den zu entfernenden Ultrafiltrationsfluß Qf vergrößerter Fluß Qd+Qf mit der physikalisch-chemischen Eigenschaft Cdo ab. In die zweite Kammer 104 strömt Blut über eine Blutzuführleitung 105 mit dem Fluß Qb und der physikalisch-chemischen Eigenschaft Cbi. Diese Kammer 104 verläßt ein Blutfluß über die Blutabführleitung 106, der um den Ultrafiltrationsfluß Qf vermindert ist und die physikalisch-chemische Eigenschaft Cbo aufweist.

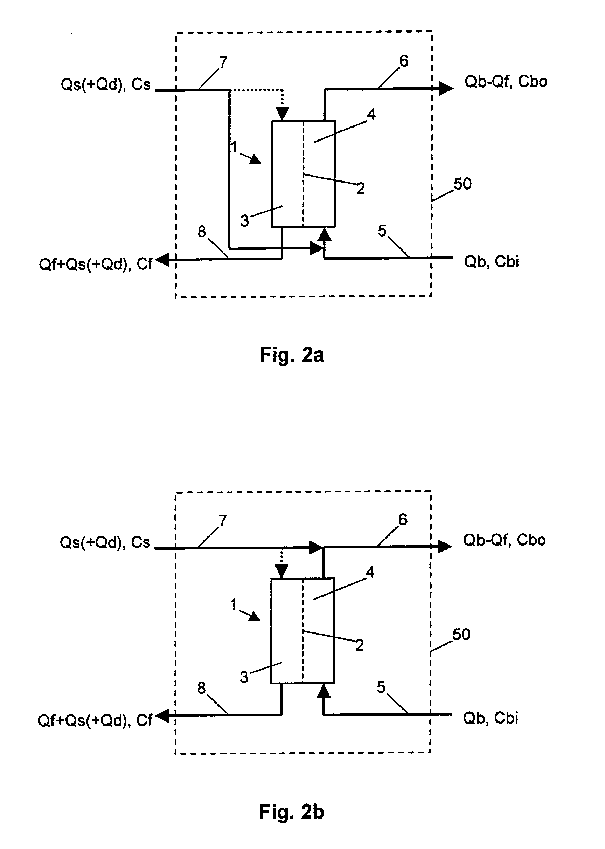

Die Fig. 2a und 2b zeigen eine entsprechend schematisierte Hämofiltrationsvorrichtung, bei der ein durch eine semipermable Membran 2 in zwei Kammern 3 und 4 unterteilter Hämofilter 1 als Blutreinigungseinheit vorgesehen ist. Bezüglich der Blutseite gelten die gleichen Begriffe wie in Fig. 1 erläutert. Weiterhin ist ein Substitutionsflüssigkeitszuführleitung 7 vorgesehen, die direkt entweder mit der Blutzuführleitung 5 (Prädilution, Fig. 2a) oder Blutabführleitung 6 (Postdilution, Fig. 2b) verbunden ist. Durch diese Leitung wird dem extrakorporalen Blutkreislauf direkt, also nicht über die Membran 2, Substitutionsflüssigkeit mit dem Fluß Qs und der physikalisch-chemischen Eigenschaft Cs zugegeben. Des weiteren wird über die Membran 2 dem Blut Flüssigkeit mit dem Fluß Qo=Qf+Qs entzogen, die in die erste Kammer 3 einströmt und diese Kammer über die Ultrafiltratabführleitung 8 mit der physikalisch-chemischen Eigenschaft Cf verläßt.

In den Fig. 2a und 2b ist des weiteren ein gepunkteter Verlauf angegeben, der von der Substitutionszuführleitung 7 abzweigt und zur ersten Kammer 3 führt. Dieser Weg wird bei einer Hämodiafiltrationsanwendung zusätzlich durchflossen. Die Flußverhältnisse ändern sich dann insofern, als die in Klammern angegebenen Terme für den Dialysierflüssigkeitfluß Qd hinzutreten. Der die Ultrafiltratabführleitung durchfließende Fluß beläuft sich dann auf Qo=Qf+Qs+Qd. Für die physikalisch-chemischen Eigenschaften Cs und Cf werden die gleichen Bezeichnungen weiterverwendet. Für die in den Fig. 2a und 2b gezeigten Verlauf bleibt Cs durch die Hämodiafiltration unverändert. Der Wert für Cf wird sich jedoch ändern, da nun Teile des Flusses Qd mit der physikalisch-chemischen Eigenschaft Cs die erste Kammer durchfließen und sich mit dem durch Membran hinzutretenden Fluß Qs+Qf vermischen, um gemeinsam über die Ultrafiltratabführleitung 8 abgeführt zu werden.

In den beiden Fig. 2a und 2b ist des weiteren ein Bereich 50 gestrichelt eingerahmt. Betrachtet man diesen Bereich als eine Art Black-Box Dialysator 1, so können die Formalismen, die sich für die in Fig. 1 gezeigte Anordnung ergeben, überraschenderweise auf die Situation der Hämofiltration übertragen werden. Falls die physikalisch-chemische Eigenschaft eine Konzentration ist, lautet insbesondere die der Gleichung 1 entsprechende Gleichung:

$$D = (Qf+Qs+Qd)\frac{Cf\text{-}Cs}{\alpha Cbi\text{-}Cs} \qquad (3).$$

Gleichung 2 geht in Gleichung 4 über:

$$D = (Qf+Qs+Qd)(1\text{-}\frac{Cf2\text{-}Cf1}{Cs2\text{-}Cs1}) = (Qf+Qs+Qd)(1\text{-}\frac{\Delta Cf}{\Delta Cs}) \qquad (4).$$

Der Begriff der Dialysance kann auf diesen Fall anschaulich übertragen werden: Er gibt den Blutfluß an, der vollständig auf das Konzentrationsniveau der Substitutionsflüssigkeit gebracht wird. Diese Konzentration Cs, vormals Cdi, stellt die maximal zu erreichende Konzentration durch die Blutreinigung dar. Des weiteren gelten die gleichen Analogie-Betrachtungen, wie sie vorher bezüglich der Harnstoff-Clearance und der Ionen-Dialysance bestanden. Dies konnte von der Anmelderin in in-vitro Versuchen, bei denen auf der Blutseite mit Harnstoff angereicherte Dialysierflüssigkeit verwendet und die Harnstoff-Clearance unabhängig direkt gemessen wurde, im Rahmen der Meßgenauigkeit bestätigt werden.

Damit können Vorrichtungen, die für den Betrieb in Hämodiatysevorrichtungen gebaut wurden und z.B. auf den in der DE 39 38 662 A1 oder DE 197 47 360 A1 genannten Methoden basieren, problemlos auch in Hämofiltrationseinrichtungen eingesetzt werden, wobei lediglich zu berücksichtigen ist, daß die richtigen Konzentrationen und Flüssigkeitsflüße verwendet werden.

Auf einen Anwendungsmodus des erfindungsgemäßen Verfahrens soll hier gesondert hingewiesen werden. Bei einem Betrieb ohne Dialysierflüssigkeit in einer Postdilutionsanordnung (Fig. 2b) scheint das erfindungsgemäße Verfahren wenig zu bewirken. Die Änderung ΔCs geht mit keiner direkten Änderung ΔCbi einher, die wiederum zu einer Änderung ΔCf führen könnte. Dann wäre ΔCf=0, wodurch D=Qf+Qs=Qo, was für diesen Fall auch zu erwarten ist, ohne daß es des eindungsgemäßen Verfahrens bedarf.

Aber selbst für diesen Fall liefert das erfindungsgemäße Verfahren bei genauer Betrachtung neue Ergebnisse, die über das erwartete Ergebnis hinausgehen (hierzu sind allerdings entsprechend genaue Sensorelemente notwendig). Wie bei der Hämodialyse, wo gezeigt werden konnte, daß eine Meßapparatur ähnlich der hier vorgestellten Anordnung die sogenannte effektive Dialysance bzw. Clearance mißt (H.D. Polaschegg, Int. J. Art. Organs 16, 185 (1993)), gelten die gleichen Effekte auch im Fall der Hämofiltration. Danach kann die Blutkonzentration Cbi durch Rezirkulationseffekte im Patienten dennoch verändert werden. Auch wenn die Auswirkungen klein sind, läßt sich damit mit dem erfindungsgemäßen Verfahren auch die effektive Dialysance für den Fall der Hämofiltration, die kleiner als die zu erwartende Dialysance D=Qo ist, bestimmen.

Bei der Prädilution kann ein ähnlicher Ausdruck für die zu erwartende Dialysance angegeben werden, der dem Umstand Rechnung trägt, daß das Blut vor dem Eintritt die zweite Kammer 4 im Verhältnis Qs/(Qb+Qs) verdünnt wurde. Aber auch hier gilt das zur Postdilution Gesagte bezüglich der effektiven Dialysance.

Die größte Bedeutung dürfte der Erfindung aber bei der Hämodiafiltration zukommen, wo das erfindungsgemäße Verfahren überhaupt erst einen Zugang zur Dialysance ermöglicht, deren zu erwartender Wert sich in diesem Fall nicht einfach aus den Flußangaben ermitteln läßt.

Bei einer Kombination von Prä- und Postdilution hat das erfindungsgemäße Verfahren außerdem den Vorteil, daß der jeweilige Anteil der Substitutionsflüssigkeit für die Einleitung vor und nach der zweiten Kammer 4 in den extrakorporalen Blutkreislauf gar nicht bekannt zu sein braucht. Das Verfahren ermittelt automatisch den richtigen Wert der Dialysance D.

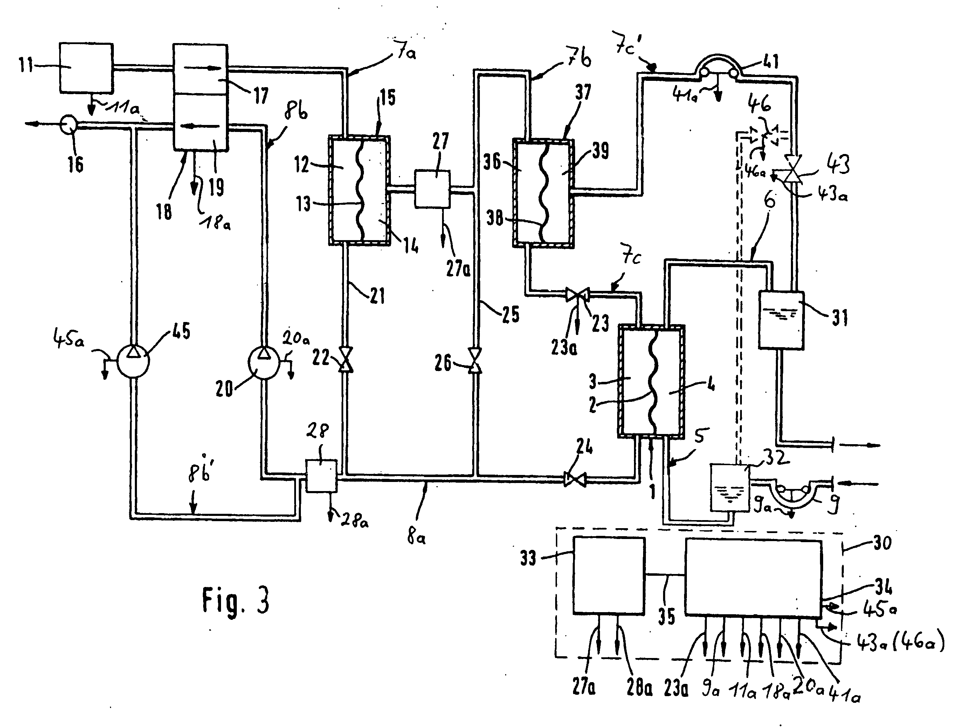

Anhand der Fig. 3 wird nun eine Ausführungsform einer erfindungsgemäßen Hämodiafiltrationsvorrichtung zur Anwendung des erfindungsgemäßen Verfahrens vorgestellt. Kernstück der Hämodiafiltrationsvorrichtung ist der Hämofilter 1, der gleichzeitig als Hämodialysator eingesetzt werden kann. Der Hämofilter 1 ist durch eine semipermeable Membran 2 in zwei Kammern 3 und 4 eingeteilt, von denen die erste Kammer 3 Teil eines Ultrafiltratabführsystems und die zweite Kammer 4 Teil eines extrakorporalen Blutkreislaufs sind.

Der extrakorporale Blutkreislauf umfaßt neben anderen, nicht näher gezeigten ge-bräuchlichen Komponenten eine Blutzuführleitung 5 mit einer Blutförderpumpe 9 und einem arteriellen Blasenfänger 32 zum Zuführen von Blut von einem Patienten zur Kammer 4 sowie eine Blutabführleitung 6 mit einem venösen Blasenfänger 31 zur Rückführung des Blutes zum Patienten.

Das Ultrafiltratabführsystem beinhaltet eine in Abschnitte 8a, 8b und 8b' unterteilte Ultrafiltratabführleitung. Der Abschnitt 8a führt aus der ersten Kammer 3 heraus, wobei ein Ventil 24 zu Absperrung dieser Ausgangsleitung des Hämofilters vorgesehen ist. Am Ende des Abschnitts 8a ist als Leitfähigkeitsmeßzelle 28 zur Erfassung der elektrischen Leitfähigkeit ausgebildetes zweites zweites Sensorelement vorgesehen, mit der die Ionenkonzentration bzw.

vorwiegend die Natriumkonzentration Cf auf bekannte Art und Weise ermittelt werden kann. Hierzu ist die Meßzelle 28 mit einer zentralen Auswerte- und Steuereinheit 30 über eine Datenleitung 28a verbunden.

Nach der Meßzelle 28 verzweigt sich die Ultrafiltratabführleitung in zwei Abschnitte 8b und 8b'. In beiden Abschnitten befindet sich je eine Förderpumpe. Im Abschnitt 8b ist dies die Dialysierflüssigkeits-/Filtratpumpe 20, an die keine besondere Genauigkeitsanforderungen gestellt werden. Sie muß lediglich eine ausreichende Förderkapazität bereitstellen, damit die erste Bilanzkammerhälfte 19 einer Bilanzkammer 18, die in den Abschnitt 8b geschaltet ist, in vorgegebenen Zeiten gefüllt werden kann. Die Bilanzkammer 18 dient der Gewährleistung, daß der Abschnitt 8b nur von einem Teil des abgeführten Ultrafiltratflusses durchflossen wird, der dem zugeführten Flüssigkeitsfluß in ein Substitutionsflüssigkeitszugabesystem (Summe aus Substitutionsflüssigkeitsfluß Qs und Dialysierflüssigkeitsfluß Qd) entspricht. Die Bilanzkammer 18 besteht dabei zweckmäßigerweise aus zwei parallel geschalteten Bilanzkammern, damit eine konstanter Fluß gewährleistet werden kann. Der Einfachheit halber wurde auf die Darstellung der zweiter Bilanzkammer sowie der diversen Eingangs- und Ausgangsventile in Fig. 3 verzichtet.

In dem Abschnitt 8b' ist eine als volumetrische, vorzugsweise als Membranpumpe ausgebildete Förderpumpe 45 vorgesehen. Mit dieser Pumpe wird der zu entfernende Ultratfiltratfluß Qf gefördert, der dem Patienten insgesamt entzogen werden soll. Die Bilanzkammer 18 sowie die Pumpen 20 und 45 sind mit entsprechenden Steuerleitungen 18a, 20a und 45a mit der Auswerte- und Steuereinheit 30 verbunden.

Die Abschnitte 8b und 8b' münden schließlich in einen Abfluß 16, wobei unerheblich ist, ob sich die beiden Abschnitte noch in der Vorrichtung wie gezeigt vereinigen oder nicht.

Frische Substitutions- und/oder Dialysierflüssigkeit wird von einer Flüssigkeitsquelle bereitgestellt, die Teil eines Substitutionsflüssigkeitzugabesystems ist. Zur Ausgestaltung der Flüssigkeitsquelle stehen dem Fachmann unterschiedliche Alternativen zur Verfügung. Neben einer Bereitstellung der fertigen Lösung in Beuteln ist dies insbesondere die Aufbereitung der Flüssigkeit in der Hämodiafiltrationsvorrichtung selbst aus Wasser und Konzentrat. Die Vorrichtung enthält hierfür diverse Meß- und Steuerelemente, auf deren Erläuterung an dieser Stelle abgesehen wird und die hinlänglich bekannt sind.

Das Substitutionsflüssigkeitzugabesystem umfaßt ferner die folgenden Komponenten: Die fertige Substitutions- und/oder Dialysierflüssigkeit fließt von der Flüssigkeitsquelle durch einen ersten Abschnitt 7a einer Substitutions-/Dialysierflüssigkeitsleitung, an den sich die Abschnitte 7b, 7c und 7c' anschließen. In den Abschnitt 7a ist die zweite Bilanzkammerhälfte 17 der Bilanzkammer 18 geschaltet. Er mündet schließlich in die erste Kammer 12 eines durch eine semipermable Membran 13 in zwei Kammern 12 und 14 geteilten ersten Sterilfilters 15. Die Flüssigkeit verläßt nach Passage der Membran 13 die zweite Kammer 14 des ersten Sterilfilters über den Abschnitt 7b der Substitutions-/Dialysierflüssigkeitsleitung, die zur ersten Kammer 36 eines durch eine semipermable Membran 38 in zwei Kammern 36 und 39 geteilten zweiten Sterilfilters 37 führt. In den Abschnitt 7b ist ein dem zweiten Sensorelement 28 entsprechendes Sensorelement 27 zur Erfassung der elektrischen Leitfähigkeit der diesen Sensor durchfließenden Flüssigkeit vorgesehen, der wiederum mit einer Datenleitung 27a mit der Auswerte- und Steuereinheit 30 verbunden ist.

Die die Membran 38 passierende Substitutionsflüssigkeit verläßt die zweite Kammer 39 des Sterilfilters 37 über den Abschnitt 7c' der Substitutionsflüssigkeitsleitung. In diesem Abschnitt ist eine Förderpumpe 41 zur Förderung des Substitutionsflüssigkeitsflusses Qs vorgesehen. Bevor der Abschnitt 7c' in den venösen Blasenfänger 31 mündet (Postdilution), ist ein Absperrventil 43 vorsehen. Alternativ oder zusätzlich kann vorgesehen sein (gestrichelt gezeichnet), den Abschnitt 7c' in den arteriellen Blasenfänger münden zu lassen (Prädilution). In diesem Abschnitt wäre dann ein weiteres Absperrventil 46 vorgesehen.

Aus der ersten Kammer 36 des zweiten Sterilfilters 37 führt ein Abschnitt 7c der Dialysierflüssigkeitsleitung zur ersten Kammer 3 des Hämofilters. Der Abschnitt 7c ist durch ein Absperrventil 23 verschließbar, daß über die Steuerleitung 23a mit der Auswerte- und Steuereinheit 30 verbunden ist. Mit diesem Ventil kann somit gesteuert werden, ob eine Hämofiltrationsbehandlung als reine Hämofiltrationsbehandlung (Ventil geschlossen) oder als Teil einer Hämodiafiltrationsbehandlung durchgeführt werden soll (Ventil geöffnet). Es ist auch möglich, während einer Behandlung den Behandlungsmodus zu wechseln.

Mit Hilfe der Ventile 43 und 46 (Steuerung über Leitungen 43a und 46a) kann gegenfalls zwischen Prä- und Postdilution gewechselt oder sogar beides gleichzeitig ermöglicht werden. Hierzu kann vorgesehen sein, die Ventile 43 und 46 zur Flußsteuerung einzusetzen oder durch eigene Fördermittel zu ergänzen/auszutauschen um die Aufteilung des Substitutionsflüssigkeitsflusses Qs zu erfassen. Für die Bestimmung des Behandlungsparameters nach dem erfindungsgemäßen Verfahren ist dies jedoch von untergeordneter Bedeutung.

Für hier nicht näher beschriebene Sicherheits- und Reinigungsfunktionen ist des weiteren eine erste Bypassleitung 21 vorgesehen, die die erste Kammer 12 des ersten Sterilfilters 15 mit dem Abschnitt 8a der Ultrafiltratabführleitung verbindet und welche durch ein Ventil 22 während des normalen Betriebs verschließbar ist. Gleiches gilt für eine zweite Bypassleitung 25, die von dem Abschnitt 7b der Substitutions-/Dialysierflüssigkeitsleitung abzweigt und aufstromig ebenfalls in den Abschnitt 8a der Ultrafiltratabführleitung mündet. Die zweite Bypassleitung ist durch ein Ventil 26 verschließbar.

Die Hämodiafiltrationsvorrichtung enthält ferner eine Auswerte- und Steuereinheit 30, die ihrerseits aus einer Auswerteeinheit 33 und einer Steuereinheit 34 besteht, die durch einen Datenleitung 35 miteinander verbunden sind. Die Steuereinheit ist über die Steuerleitungen 9a, 11a, 18a, 20a, 23a, 41a, 43a, 45a und 46a mit den diversen Steuerelementen der Hämodiafiltrationsvorrichtung verbunden, um deren Betrieb steuern zu können. Dabei wurden nur die Steuerelemente/-leitungen wähnt, die für das Verständnis der Erfindung erforderlich sind.

Die Auswerteeinheit ist über Datenleitungen mit einigen Sensorelementen verbunden. Im vorliegenden Fall sind dies im besonderen die beiden Leitfähigkeitssensoren 27 und 28.

Die Ausgestaltung der erfindungsgemäßen Vorrichtung, bei der eine erste Bilanziereinrichtung (17) in der Substitutions-/Dialysierflüssigkeitsleitung (7a, 7b, 7c', 7c) und/oder eine zweite Bilanziereinrichtung (19, 45) in der Ultrafiltratabführleitung vorgesehen sind, hat den Vorteil, daß mit diesen Einrichtungen gleichzeitig der für die Anwendung von Gleichung 4 relevante abgeführte Fluß Qo=Qs+Qf+Qd bestimmt werden kann. Bei der Ausführungsform nach Fig. 3 ist das Volumen einer Bilanzkammerfüllung sehr genau bekannt. Über die Frequenz der Bilanzkammertakte kann der Fluß Qs+Qd sehr genau bestimmt werden. Die Pumpe 45 ist volumetrisch und kann damit ebenfalls genutzt werden, um - wie hier als Membranpumpe über die Frequenz der Pumphübe und das bekannte Hubvolumen - den Fluß Qf zu bestimmen. Dies beseitigt Ungenauigkeiten, die z.B. durch eine als Rollenpumpe gestaltete Substitutionsflüssigkeitspumpe 41 entstehen, deren Fördermenge aufgrund von Toleranzschwankungen des Pumpschlauchsegments und auch durch Ladedruckschwankungen in einem gewissen Bereich schwanken kann.

Zur Anwendung des erfindungsgemäßen Verfahrens geht die Auswerte- und Steuereinheit 30 die folgenden Verfahrensschritte durch: Die Einrichtung 11 wird so angesteuert, daß sie Substitutionsflüssigkeit mit einer Konzentration Cs1 bereitstellt. Diese Konzentration wird über den ersten Meßsensor 27 aufgezeichnet und an die Auswerteeinheit 33 übermittelt. An den Fördereinrichtungen/Pumpen 9, 18, 20, 41 und 45 werden die Flüssigkeitsflüsse Qb, Qs, Qf und Qd eingestellt sowie die Ventile 23, 43 und 46 je nach Betriebsart geöffnet oder geschlossen. Die Werte für Qb, Qs, Qf und Qd werden außerdem von der Steuereinheit 34 an die Auswerteeinheit 33 übermittelt. Die Konzentrationswerte Qf1 werden durch den zweiten Meßsensor 28 aufgezeichnet und an die Auswerteeinheit 33 übermittelt.

Zu einem Zeitpunkt, an dem der Steuerablauf dies automatisiert vorsieht oder dies aus einem anderen Grund, z.B. manuell veranlaßt wurde, führt die Einrichtung 11 auf Anweisung der Steuereinheit 34 eine Änderung der Natriumkonzentration der Substitutionsflüssigkeit z.B. in Bolusform durch, d.h. dieNatriumkonzentration wird kurzzeitig verändert und nimmt danach wieder den Ausgangswert an (bei einer Bereitstellung der Substitutionsflüssigkeit in Form von Beuteln kann dies über eine nicht dargestellte automatisierte oder manuelle Injektionsstelle in die Substitutionsflüssigkeitsleitungsabschnitt 7a geschehen). Die entsprechenden Konzentrationen Cs2 und Cf2 werden aufgezeichnet und an die Auswerteeinheit 33 übermittelt. Nach dem Abklingen des Bolus bestimmt die Auswerteeinheit 33 als Behandlungsparameter die Ionen-Dialysance bzw. die Harnstoff-Clearance der Hämodiafiltrationsvorrichtung, in dem zuerst die integrierten Differenzen ΔCs und ΔCf zwischen den ursprünglichen Konzentrationen Cs1 und Cf1 und den während des Bolus geänderten Konzentrationen Cs2 und Cf2 bestimmt werden und danach die Ionen-Dialysance und damit die Harnstoff-Clearance mit Hilfe von Gleichung 4 bestimmt wird. Dieser Wert kann dann über eine nicht gezeigte Anzeigeeinheit, die meist sowie Teil derartiger Blutbehandlungsvorrichtungen ist, angezeigt werden.

Die Messungen können dabei in vorgegebenen Intervallen automatisch wiederholt werden (z.B. 30 min), um damit die Harnstoff-Clearance über den Verlauf der gesamten Hämofiltrationsbehandlung bestimmen zu können. Durch Integration über der Zeit kann die Auswerteeinheit 33 den Parameter Kt ermitteln, der entweder allein oder mit Hilfe eines zuvor eingegebenen oder anderweitig bestimmten Wertes für das Verteilungsvolumen V in den Behandlungseffizienzparameter Kt/V umgerechnet und angezeigt wird. Die ermittelten Größen können natürlich auch dazu dienen, den weiteren Behandlungsverlauf durch die Steuereinheit 34 so anzupassen, daß ein angestrebtes Behandlungsziel auch wirklich erreicht werden kann.

Gemäß einer besonders vorteilhaften Weiterentwicklung der Erfindung ist die Hämodiafiltrationsvorrichtung auch in der Lage, nach eine anfänglichen Bestimmung der Ionen-Dialysance D weitere Werte der Dialysance für spätere Zeitpunkte zu berechnen, bei denen sich mindestens einer der Flüsse Qs, Qf, Qd oder Qb geändert hat. Bei einer routinemäßigen Messung werden Ionen in das Blut verschoben, so daß derartige Messungen - auch wenn sie gegenseitig durch positive und negative Boli kompensiert sein können und einzeln keine Auswirkungen auf den Patienten haben - nicht zu oft durchgeführt werden sollten. Dies ist auch daher sinnvoll, daß ansonsten während einer Messung, die einige Minuten in Anspruch nehmen kann, die Variationsmöglichkeiten von anderen Behandlungsparametern eingeschränkt sein kann. Aus einem ähnlichem Grund ist auch ein Verfahren für die Hämodialyse bekannt geworden, das nur durch Flußänderungen verursachte Dialysanceänderungen bestimmen kann, ohne daß es der erneuten Messung bedarf. Dieses Verfahren ist in der EP 1 062 960 A2 der Anmelderin beschrieben, auf die ausdrücklich Bezug genommen wird.

Es ist daher anstrebenswert, eine derartige Vereinfachung auch für die Bestimmung der Dialysance bei der Hämofiltration zur Verfügung zu haben. Die Weiterbildung der Erfindung sieht daher vor, daß die Auswerte- und Steuereinheit 30 zwischen zwei Messungen der Dialysance in der Lage ist, bei einer registrierten Änderung der Flußverhältnisse automatisch einen neuen Diatysance-Wert zu bestimmen, ohne eine Messung herbeiführen zu müssen. Hierzu wertet die Auswerteeinheit 33 ständig die über die Leitung 35 übermittelten Werte für Qf, Qs, Qd und Qb aus.

Sobald sich eine Änderung gegenüber von einem vorher ermittelten ersten Satz Qf1, Qs1, Qd1 und Qb1 ergibt, für den eine Dialysance D1 bestimmt und abgespeichert wurde, bestimmt die Auswerteeinheit 33 eine neue Dialysance D2 für den neuen, zweiten Satz Qf2, Qs2, Qd2 und Qb2. Die Auswerteeinheit 33 geht dabei wie folgt vor:

Mit Hilfe der Gleichung 5 bestimmt die Auswerteeinheit 33 zunächst den diffusiven Anteil der Dialysance D1:

$$D1diff = \frac{Qb1 + \kappa Qs1}{Qb1 - Qf1 - (1-\kappa)Qs1}\left(\frac{Qb1 + \kappa Qs1}{Qb1}D1 - Qf1 - Qs1\right) \tag{5},$$

wobei K=1 bei Prädilution und K=0 bei Postdilution. Daraufhin wird der Filterkoeffizient k0A bestimmt, der als konstant zwischen den beiden Zeitpunkten 1 und 2 angenommen wird:

$$k0A = \frac{(Qb1 + \kappa Qs1)Qd1}{Qd1 - Qb1 - \kappa Qs1} \text{ in } \frac{\frac{D1diff}{Qd1} - 1}{\frac{D1diff}{Qb1 + \kappa Qs1} - 1} \tag{6}.$$

Mit diesem Ausdruck für k0A kann nun die diffusive Dialysance D2diff für den zweiten Satz von Flüssen nach Gleichung 7 bestimmt werden:

$$D2diff = Qb2\frac{e^{\gamma} - 1}{e^{\gamma} - \frac{Qb2}{Qd2}} \text{ , mit } \gamma = k0A\frac{Qd2 - Qb2}{Qb2\,Qd2} \tag{7}.$$

D2diff kann nun in Gleichung 5 eingesetzt werden, welche nach Austauschen des ersten Qb1, Qs1, Qf1 mit dem zweiten Satz Qb2, Qs2, Qf2 nach D2 aufgelöst werden kann.

Die Erfindung ermöglicht damit eine einfache und unkompliziertes Verfahren zur Bestimmung eines Behandlungsparameters während der Hämofiltration und Hämodiafiltration, wobei auf ein in der Hämodialyse bewährtes Verfahren zurückgegriffen werden kann. Eine entsprechendes Hämo(dia)filtrationsgerät braucht nur geringfügig modifiziert zu werden, damit das Verfahren automatisiert angewendet werden kann.

Bezugszeichenliste

1    Hämofilter
2    semipermeable Membran des Hämofilters
3    erste Kammer des Hämofilters
4    zweite Kammer des Hämofilter
5    Blutzuführleitung
6    Blutabführleitung
7a   erster Abschnitt der Dialysier-/Substitutionsflüssigkeitsleitung
7b   zweiter Abschnitt der Dialysier-/Substitutionsflüssigkeitleitung
7c   dritter Abschnitt der Dialysierflüssigkeitsleitung
7c'  dritter Abschnitt der Substitutionsleitung
8a   erster Abschnitt der Ultrafiltratabführleitung
8b   zweiter Abschnitt der Ultrafiltratabführleitung für 1:1 Bilanzierung
8b'  zweiter Abschnitt der Ultrafiltratabführleitung für Flüssigkeitsentzug
9    Blutpumpe
11   Dialysier-/Substitutionsflüssigkeitsquelle
12   erste Kammer des ersten Sterilfilters
13   semipermeable Membran des ersten Sterilfilters
14   zweite Kammer des ersten Sterilfilters
15   erster Sterilfilter

| 16 | Abfluß |
|---|---|
| 17 | zweite Bilanzkammerhälfte |
| 18 | Bilanzkammer |
| 19 | erste Bilanzkammerhälfte |
| 20 | Dialysierflüssigkeit-/Ultrafiltratumwälzpumpe |
| 21 | erste Bypass-Leitung |
| 22 | erstes Bypass-Ventil |
| 23 | Dialysierflüssigkeitszuführventil |
| 24 | Ultrafiltratabführventil |
| 25 | zweite Bypass-Leitung |
| 26 | zweites Bypass-Ventil |
| 27 | Meßzelle zur Erfassung der Leitfähigkeit der Substitutionsflüssigkeit |
| 28 | Meßzelle zur Erfassung der Leitfähigkeit der in der Ultrafiltratabführleitung befindlichen Flüssigkeit |
| 30 | Auswerte- und Steuereinheit |
| 31 | venöser Blasenfänger |
| 32 | arterieller Blasenfänger |
| 33 | Auswerteeinheit |
| 34 | Steuereinheit |
| 35 | Datenleitung zwischen Auswert- und Steuereinheit |
| 36 | erste Kammer des ersten Sterilfilters |
| 37 | zweites Sterilfilter |
| 38 | semipermeable Membran des zweiten Sterilfilters |
| 39 | zweite Kammer des zweiten Sterilfilters |
| 41 | Substitutionsflüssigkeitspumpe |
| 43 | Postdilutionventil |
| 45 | Pumpe für Flüssigkeitsentzug |
| 46 | Prädilutionsventil |

## Patentansprüche

1. Verfahren zur Bestimmung von Behandlungsparametern an einer Hämofiltrationsvorrichtung,
mit einem durch eine semipermeable Membran (2) in zwei Kammern geteilten Blutreinigungselement (1), deren erste Kammer (3) Teil eines Ultrafiltratabführsystems und deren zweite Kammer (4) Teil eines extrakorporalen Blutkreislaufs ist,
mit einem Substitutionsflüssigkeitszugabesystem (7; 7a, 7b, 7c', 11, 17, 41), mit dem Substitutionsflüssigkeit dem extrakorporalen Blutkreislauf zugegeben werden kann,
wobei der aus der ersten Kammer (3) abgeführte Flüssigkeitsfluß Qo bestimmt wird,
wobei eine physikalisch-chemische Eigenschaft Cs1 in der Substitutionsflüssigkeit und die entsprechende physikalisch-chemische Eigenschaft Cf1 in der abgeführten Flüssigkeit bestimmt werden,
wonach die physikalisch-chemische Eigenschaft Cs in der Substitutionsflüssigkeit verändert wird,
wobei die physikalisch-chemische Eigenschaft Cs2 in der Substitutionsflüssigkeit und die entsprechende physikalisch-chemische Eigenschaft Cf2 in der abgeführten Flüssigkeit erneut bestimmt werden,
wonach ein Behandlungsparameter aus den ermittelten Größen des abgeführten Flüssigkeitsfluß Qo und den physikalisch-chemischen Eigenschaften Cs1 und Cs2 in der Substitutionsflüssigkeit und Cf1 und Cf2 in der abgeführten Flüssigkeit ermittelt wird.

2. Verfahren nach Anspruch 1, **dadurch gekennzeichnet, daß** die Veränderung der physikalisch-chemischen Eigenschaft Cs stufen- oder bolusförmig ist.

3. Verfahren nach Anspruch 2, **dadurch gekennzeichnet, daß** der Behandlungsparameter die Dialysance des Blutreinigungselementes ist und nach dem folgenden Ausdruck, mit $\Delta Cf = Cf2 - Cf1$ und $\Delta Cs = Cs2 - Cs1$, bestimmt wird:

$$D = (1 - \frac{\Delta Cf}{\Delta Cs}) \cdot Qo.$$

4. Verfahren nach einem der vorhergehenden Ansprüche, **dadurch gekennzeichnet, daß** der Behandlungsparameter die Bluteingangskonzentration Cbi ist.

5. Verfahren nach einem der vorhergehenden Ansprüche, **dadurch gekennzeichnet, daß** der dem extrakorporalen Kreislauf insgesamt entzogene Ultrafiltratfluß Qf und der Substitutionsflüssigkeitsfluß Qs bestimmt werden und aus der Summe beider Größen der abgeführte Fluß Qo bestimmt wird.

6. Verfahren nach einem der Ansprüche 1 bis 4, **dadurch gekennzeichnet, daß** zusätzlich eine Zuführung von Dialysierflüssigkeit mit einem Fluß Qd in die erste Kammer (3) vorgesehen ist und daß der abgeführte Fluß Qo als Summe des dem extrakorporalen Kreislauf insgesamt entzogenen Ultrafiltratflußes Qf, des Substitutionsflüssigkeitsflußes Qs und des zugeführten Flußes Qd bestimmt wird.

7. Verfahren nach einem der Ansprüche 1 bis 6, **dadurch gekennzeichnet, daß** zusätzlich der Blutfluß Qb als effektiven Blutflüssigkeitsfluß, der in die Blutzuführleitung einströmt und in dem sich die Änderung der physikalisch-chemischen Eigenschaft auswirken kann, bestimmt wird.

8. Verfahren nach Anspruch 7, **dadurch gekennzeichnet, daß** die Dialysance D1 bei einem ersten Satz von Flüssen Qd1, Qb1, Qs1 und Qf1 bestimmt wird und daraufhin die Dialysance D2 für einen zweiten Satz von Flüssen Qd2, Qb2, Qs2 und Qf2 auf der Basis der Werte des ersten Satzes von Flüssen und D1 errechnet wird.

9. Verfahren nach Anspruch 8, **dadurch gekennzeichnet, daß** auf der Grundlage von mehreren Dialysancewerten D1i, die zu verschiedenen Zeitpunkten i während einer Hämofiltrationsbehandlung ermittelt wurden, und mehreren Dialysancewerten D2j, die für verschiedene Zeitpunkte j während einer Hämofiltrationsbehandlung errechnet wurden, die insgesamt erfolgte Behandlungseffizienz Kt durch eine Summenbildung der jeweiligen Dialysancewerte multipliziert mit der Zeitdifferenz zur vorherigen bzw. nachfolgenden Messung/Errechnung bestimmt wird.

10. Hämofiltrationsvorrichtung zur Durchführung des Verfahrens nach Anspruch 1,
mit einem durch eine semipermeable Membran (2) in zwei Kammern geteilten Blutreinigungselement (1), deren erste Kammer (3) Teil eines Ultrafiltratabführsystems und deren zweite Kammer (4) Teil eines extrakorporalen Blutkreislaufs ist,
wobei weitere Teile des extrakorporalen Blutkreislaufs eine Blutzuführleitung (5), die zu der zweiten Kammer (4) führt, und eine Blutabführleitung (6), die Blut von der zweiten Kammer (4) abführt, umfassen,
wobei weitere Teile des Ultrafiltratabführsystems eine Ultrafiltratabführleitung (8; 8a, 8b, 8b'), die die Flüssigkeit aus der ersten Kammer (3) abführt und eine Ultrafiltratfördervorrichtung (20, 45) zum gezielten Flüssigkeitsentzug eines Abführflusses Qo durch die Membran (2) über die Ultrafiltratabführleitung (8; 8a, 8b, 8b') umfassen,
mit einem Substitutionsflüssigkeitszugabesystem, das eine Substitutionsflüssigkeitsleitung (7; 7a, 7b, 7c'), die von einer Quelle zur Bereitstellung von Substitutionsflüssigkeit zum extrakorporalen Blutkreislauf verläuft, und eine Substitutionsfördervorrichtung (41) zum Fördern von Substitutionsflüssigkeit in der Substitutionsflüssigkeitsleitung (7; 7a, 7b, 7c') umfaßt,
mit einem an der Substitutionsflüssigkeitsleitung (7; 7a, 7b, 7c') angeordneten ersten Sensorelement (27) zur Bestimmung einer physikalisch-chemischen Eigenschaft Cs der Substitutionsflüssigkeit,
mit einem an der Ultrafiltratabführleitung (8; 8a, 8b, 8b') angeordneten zweiten Sensorelement (28) zur Bestimmung der entsprechenden physikalisch-chemischen Eigenschaft Cf der abgeführten Flüssigkeit,
**dadurch gekennzeichnet,**
**daß** eine Einrichtung (11) zur Änderung der physikalisch-chemischen Eigenschaft Cs der Substitutionsflüssigkeit vorgesehen ist,
**daß** eine Auswerte- und Steuereinheit (30) vorgesehen ist, die zunächst die Meßwerte Cs1 und Cf1 des ersten und zweiten Sensorelementes aufzeichnet, dann die Einrichtung (11) zur Änderung der physikalisch-chemischen Eigenschaft der Substitutionsflüssigkeit ansteuert, um eine solche Änderung herbeizuführen, die aufgrund der Änderung geänderten Meßwerte Cs2 und Cf2 des ersten und zweiten Sensorelementes erneut aufzeichnet, und aus den abgespeicherten Werten (Cs1, Cs2, Cf1, Cf2, Qo) den Behandlungsparameter bestimmt.

11. Hämofiltrationsvorrichtung nach Anspruch 10, **dadurch gekennzeichnet, daß** die Auswerte- und Steuereinheit (30) die Einrichtung (11) zur Änderung der physikalisch-chemischen Eigenschaft der Substitutionsflüssigkeit derart ansteuert, daß die Änderung stufen- oder bolusförmig ist.

12. Hämofiltrationsvorrichtung nach Anspruch 11, **dadurch gekennzeichnet, daß** der Behandlungsparameter die Dialysance des Blutreinigungselementes ist und daß die Auswerte- und Steuereinheit (30) geeignet ist, die Dialysance nach dem folgenden Ausdruck zu bestimmen, mit $\Delta Cf = Cf2 - Cf1$ und $\Delta Cs = Cs2 - Cs1$:

$$D = (1 - \frac{\Delta Cf}{\Delta Cs}) \cdot Qo.$$

13. Hämofiltrationsvorrichtung nach einem der Ansprüche 10 bis 12, **dadurch gekennzeichnet, daß** der Behandlungsparameter die Bluteingangskonzentration Cbi ist.

14. Hämofiltrationsvorrichtung nach einem der Ansprüche 10 bis 13, **dadurch gekennzeichnet, daß** die Auswerte- und Steuereinheit (30) geeignet ist, Stellund/oder Meßwerte für den insgesamt dem extrakorporalen Kreislauf zu entziehendem Ultrafiltratfluß Qf und dem Substituatflüssigkeitsfluß Qs abzuspeichern und als Summe dieser beiden Flüsse den Abführungsfluß Qo zu bestimmen.

15. Hämofiltrationsvorrichtung nach einem der Ansprüche 10 bis 13, **dadurch gekennzeichnet, daß** die Quelle (11) zur Bereitstellung von Substitutionsflüssigkeit auch eine Quelle zur Bereitstellung von Dialysierflüssigkeit ist, von der eine Dialysierflüssigkeitszuführleitung (7; 7a, 7b ,7c) zur ersten Kammer (3) führt.

16. Hämofiltrationsvorrichtung nach Anspruch 15, **dadurch gekennzeichnet, daß** die Auswerte- und Steuereinheit (30) geeignet ist, den Abführungsfluß Qo als Summe von Stell- und/oder Meßwerten für den insgesamt dem extrakorporalen Kreislauf zu entziehendem Ultrafiltratfluß Qf, dem Substituatflüssigkeitsfluß Qs und dem in die erste Kammer (3) fließenden Dialysierflüssigkeitsfluß Qd abzuspeichern.

17. Hämofiltrationsvorrichtung nach Anspruch 15 oder 16, **dadurch gekennzeichnet, daß** die Substitutionsflüssigkeitzuführleitung und die Dialysierflüssigkeitszuführleitung in einem ersten Bereich als gemeinsame Leitung (7a, 7b) und nach einer Verzweigung in einem zweiten Bereich als getrennte Leitung (7c', 7c) ausgebildet sind.

18. Hämofiltrationsvorrichtung nach einem der Ansprüche 10 bis 17, **dadurch gekennzeichnet, daß** in der Substitutionsleitung (7; 7a, 7b, 7c') eine erste Bilanziereinrichtung (17) und in der Ultrafiltratabführleitung (8; 8a, 8b, 8b') eine zweite Bilanziereinrichtung (18, 45) vorgesehen sind.

19. Hämofiltrationsvorrichtung nach Anspruch 18, **dadurch gekennzeichnet, daß** sich die Ultrafiltratabführleitung in eine erste Leitung (8b) und eine zweite Leitung (8b') verzweigt, wobei die erste Leitung (8b) durch eine als erste Bilanzkammerhälfte (19) ausgebildeten ersten Teil der zweiten Bilanziereinrichtung verläuft, in der zweiten Leitung (8b') eine Einrichtung (45) zur Förderung des insgesamt dem extrakorporalen Kreislauf zu entziehendem Ultrafiltratfluß Qf als zweiter Teil der zweiten Bilanziereinrichtung vorgesehen ist und die Substitutionsleitung (7; 7a) durch eine als zweite Bilanzkammerhälfte (17) ausgebildete erste Bilanziereinrichtung verläuft.

20. Hämofiltrationsvorrichtung nach Anspruch 18 oder 19, **dadurch gekennzeichnet, daß** die erste (17) und/oder zweite (19) Bilanziereinrichtung (18) so ausgebildet ist, daß der Fluß Qs+Qd bestimmbar und durch die Auswerte- und Steuereinheit (30) erfaßbar ist.

21. Hämofiltrationsvorrichtung nach Anspruch 18, 19 oder 20, **dadurch gekennzeichnet, daß** die erste (17) und/oder zweite (19, 45) Bilanziereinrichtung so ausgebildet sind, daß aus den sie durchfließenden Flüssen der insgesamt dem extrakorporalen Kreislauf zu entziehende Ultrafiltratfluß Qf bestimmbar und durch die Auswerte- und Steuereinheit (30) erfaßbar ist.

22. Hämofiltrationsvorrichtung nach einem der Ansprüche 10 bis 21, **dadurch gekennzeichnet, daß** die Substituatflüssigkeitzuführleitung (7c') in die Blutabführleitung (6) mündet (Postdilution).

23. Hämofiltrationsvorrichtung nach einem der Ansprüche 10 bis 21, **dadurch gekennzeichnet, daß** die Substituatflüssigkeitszuführleitung (7c') in die Blutzuführleitung (5) mündet (Prädilution).

24. Hämofiltrationvorrichtung nach einem der Ansprüche 10 bis 23, **dadurch gekennzeichnet, daß** die physikalisch-chemische Eigenschaft eine Konzentration, insbesondere die Natriumkonzentration ist

25. Hämofiltrationsvorrichtung nach Anspruch 24, **dadurch gekennzeichnet, daß** die ersten und zweiten Sensorelemente Leitfähigkeitssensoren sind.

26. Hämofiltrationsvorrichtung nach einem der Ansprüche 10 bis 25, **dadurch gekennzeichnet, daß** die Auswerte- und Steuereinheit (30) geeignet ist, den Blutfluß Qb als dem effektiven Flüssigkeitsfluß, der in die Blutzuführleitung (5) einströmt und in dem sich die Änderung der physikalisch-chemischen Eigenschaft auswirken kann, abzuspeichern.

27. Hämofiltrationsvorrichtung nach Anspruch 26, **dadurch gekennzeichnet, daß** die Auswerte- und Steuereinheit (30) geeignet ist, die Dialysance D1 bei einem ersten abgespeicherten Satz von Flüssen Qd1, Qb1, Qs1 und Qf1 zu bestimmen und abzuspeichern und auf der Grundlage dieser Werte die Dialysance D2 für einen zweiten Satz von Flüssen Qd2, Qb2, Qs2 und Qf2 zu errechnen.

28. Hämofiltrationsvorrichtung nach Anspruch 27, **dadurch gekennzeichnet, daß** die Auswerte- und Steuereinheit (30) geeignet ist, auf der Grundlage von mehreren abgespeicherten Dialysancewerten D1i, die zu verschiedenen Zeitpunkten i während einer Hämofiltrationsbehandlung ermittelt wurden, und mehreren abgespeicherten Dialysancewerten D2j, die für verschiedene Zeitpunkte j während einer Hämofiltrationsbehandlung errechnet wurden, die insgesamt erfolgte Behandlungseffizienz Kt durch eine Summenbildung der jeweiligen Dialysancewerte multipliziert mit der Zeitdifferenz zur vorherigen bzw. nachfolgenden Messung/Errechnung zu bestimmen.

29. Hämofiltrationsvorrichtung nach einem der Ansprüche 10 bis 28, **dadurch gekennzeichnet, daß** die Hämofiltrationsvorrichtung ferner eine Anzeigeeinrichtung umfaßt, auf der der Behandlungsparameter, insbesondere die Dialysance D, Zwischenergebnisse und/oder die erfolgte Behandlungseffizienz Kt anzeigbar sind.

**Claims**

1. A method for determining treatment parameters on a haemofiltration device,
   comprising a blood cleansing element (1) divided into two chambers by a semipermeable membrane (2), with the first chamber (3) forming part of an ultrafiltrate outlet system and with the second chamber (4)forming part of an extracorporeal blood circulation system;
   comprising a substitution-fluid addition system (7; 7a, 7b, 7c', 11, 17, 41) with which substitution fluid can be added to the extracorporeal blood circulation system;
   wherein the fluid flow Qo, led away from the first chamber (3), is determined;
   wherein a physical-chemical characteristic Cs1 in the substitution fluid and the corresponding physical-chemical characteristic Cf1 in the fluid led away are determined;
   according to which the physical-chemical characteristic Cs in the substitution fluid is changed;
   wherein the physical-chemical characteristic Cs2 in the substitution fluid and the corresponding physical-chemical characteristic Cf2 in the fluid led away are determined anew;
   according to which a treatment parameter is determined in the fluid led away, from the determined values of the fluid flow Qo led away, and the physical-chemical characteristics Cs1 and Cs2 in the substitution fluid and Cf1 and Cf2.

2. The method according to claim 1, **characterised in that** the change in the physical-chemical characteristic Cs is step-shaped or bole-shaped.

3. The method according to claim 2, **characterised in that** the dialysance of the blood cleansing element is the treatment parameter; it is determined according to the following expression, with $\Delta Cf = Cf2-Cf1$ and $\Delta Cs = Cs2-Cs1$:

$$D = \left(1 - \frac{\Delta Cf}{\Delta Cs}\right) \cdot Qo.$$

4. The method according to one of the preceding claims **characterised in that** the blood input concentration Cbi is the treatment parameter.

5. The method according to one of the preceding claims, **characterised in that** the ultrafiltrate flow Qf and the substitution fluid flow Qs removed in total from the extracorporeal circulation system are determined and from the sum of the two values, the removed flow Qo is determined.

6. The method according to one of claims 1 to 4, **characterised in that** in addition, admission of dialysis fluid at a flow Qc into the first chamber (3) is provided, and **in that** the removed flow Qo is determined as the sum of the ultrafiltrate flow Qf removed in total from the extracorporeal circulation system, the substitution fluid flow Qs and the added flow Qd.

7. The method according to one of claims 1 to 6, **characterised in that** in addition the blood flow Qb is determined as the effective blood plasma flow which flows into the blood inlet pipe and in which the change in the physical-chemical characteristic can have an effect.

8. The method according to claim 7, **characterised in that** the dialysance D1 is determined in a first set of flows Qd1, Qb1, Qs1 and Qf1 and subsequently the dialysance D2 is calculated for a second set of flows Qd2, Qb2, Qs2 and Qf2, based on the values of the first set of flows and D1.

9. 'The method according to claim 8, **characterised in that** based on several dialysance values D1i, which were determined at various points in time i during haemofiltration treatment, and several dialysance values D2j which were calculated at various points in time j during haemofiltration treatment, the total treatment efficiency Kt achieved is determined by summation of the corresponding dialysance values, multiplied by the time difference to the previous or subsequent instance of measuring/calculation.

10. A haemofiltration device for carrying out the method according to claim 1,
    comprising a blood cleansing element (1) divided into two chambers by a semipermeable membrane (2), with the first chamber (3) forming part of an ultrafiltrate outlet system and with the second chamber (4) forming part of an extracorporeal blood circulation system;
    wherein further parts of the extracorporeal blood circulation system comprise a blood inlet pipe (5) which leads to the second chamber (4), and a blood outlet pipe (6) which removes blood from the second chamber (4);
    wherein further parts of the ultrafiltrate outlet system comprise an ultrafiltrate outlet pipe (8; 8a, 8b, 8b') which removes the fluid from the first chamber (3), and an ultrafiltrate conveyance device (20, 45) for targeted fluid removal from a removal flow Qo through the membrane (2) via the ultrafiltrate outlet pipe (8; 8a, 8b, 8b');
    comprising a substitution fluid addition system comprising a substitution fluid pipe (7; 7a, 7b, 7c') which leads from a source (11) for the provision of substitution fluid to the extracorporeal blood circulation system, and a substitution conveyance device (41) for conveying substitution fluid in the substitution fluid pipe (7; 7a, 7b, 7c');
    comprising a first sensor element (27) arranged on the substitution fluid pipe (7; 7a, 7b, 7c') for determining a physical-chemical characteristic Cs of the substitution fluid;
    comprising a second sensor element (28) arranged on the ultrafiltrate outlet pipe (8; 8a, 8b, 8b') for determining the corresponding physical-chemical characteristic Cf of the fluid led away;
    **characterised in that**
    a device (11) for changing the physical-chemical property Cs of the substitution fluid is provided;
    that an evaluation and control unit (30) is provided which first records the measured values Cs1 and Cf1 of the first and second sensor elements; then controls the device (11) for changing the physical-chemical characteristic of the substitution fluid so as to bring about such a change; which again records the measured values Cs2 and Cf2 of the first and second sensor element, said values having changed because of the change; and which determines the treatment parameter from the saved values (Cs1, Cs2, Cf1, Cf2, Qo).

11. The haemofiltration device according to claim 10, **characterised in that** the evaluation and control unit (30) controls the device (11) for changing the physical-chemical characteristic of the substitution fluid such that the change is step-shaped or bole-shaped.

12. The haemofiltration device according to claim 11, **characterised in that** the dialysance of the blood cleansing element is the treatment parameter, and that the evaluation and control unit (30) is suitable for determining the dialysance according to the following expression, with ΔCf=Cf2-Cf1 and ΔCs =Cs2-Cs1:

$$D = \left(1 - \frac{\Delta Cf}{\Delta Cs}\right) \cdot Qo.$$

13. The haemofiltration device according to one of claims 10 to 12, **characterised in that** the blood input concentration Cbi is the treatment parameter.

14. The haemofiltration device according to one of claims 10 to 13, **characterised in that** the evaluation and control unit (30) is suitable for saving set values and/or measured values for the total ultrafiltrate flow Qf to be removed from the extracorporeal blood circulation system, and the substitute-fluid flow Qs, and as a sum of these two flows for determining the removal flow Qo.

15. The haemofiltration device according to one of claims 10 to 13, **characterised in that** the source (11) for providing substitution fluid is also a source for providing dialysis fluid, from which source (11) a dialysis fluid inlet pipe (7; 7a, 7b, 7c) leads to the first chamber (3).

16. The haemofiltration device according to claim 15, **characterised in that** the evaluation and control unit (30) is suitable for saving the removal flow Qo as the sum of set values and/or measured values for the total ultrafiltrate flow Qf to be removed from the extracorporeal blood circulation system, the substitute-fluid flow Qs, and the dialysis fluid flow Qd flowing into the first chamber (3).

17. The haemofiltration device according to claim 15 or 16, **characterised in that** the substitution fluid inlet pipe and the dialysis fluid inlet pipe in a first section are a common pipe (7a, 7b) and after branching off, in a second section are separate pipes (7c', 7c).

18. The haemofiltration device according to one of claims 10 to 17, **characterised in that** in the substitution pipe (7; 7a, 7b, 7c') a first balancing device (17) is provided and in the ultrafiltrate outlet system (8; 8a, Sb, 8b') a second balancing device (18, 45) is provided.

19. The haemofiltration device according to claim 18, **characterised in that** the ultrafiltrate outlet pipe branches into a first pipe (8b) and a second pipe (8b'), wherein the first pipe (8b) passes through a first part of the second balancing device, said first part being designed as a first half (19) of the second balancing chamber; **in that** in the second pipe (8b') a device (45) for conveying the total ultrafiltrate flow Qf to be removed from the extracorporeal circulation system as the second part of the second balancing device is provided; and **in that** the substitution pipe (7; 7a) passes through a first balancing device which is designed as a second half (17) of the balancing chamber.

20. The haemofiltration device according to claim 18 or 19, **characterised in that** the first (17) and/or second (19) balancing device (18) are/is designed such that the flow Qs+Qd can be determined and can be acquired by the evaluation and control unit (30).

21. The haemofiltration device according to claim 18, 19 or 20, **characterised in that** the first (17) and/or second (19, 45) balancing device is/are designed so that from the flows flowing through it, the total ultrafiltrate flow Qf to be removed from the extracorporeal circulation system can be determined and can be acquired by the evaluation and control unit (30).

22. The haemofiltration device according to one of claims 10 to 21, **characterised in that** the substitute-fluid inlet pipe (7c') leads into the blood outlet pipe (6) (post-dilution).

23. The haemofiltration device according to one of claims 10 to 21, **characterised in that** the substitute-fluid inlet pipe (7c') leads into the blood inlet pipe (5) (pre-dilution).

24. The haemofiltration device according to one of claims 10 to 23, **characterised in that** the physical-chemical characteristic is a concentration, in particular the sodium concentration.

25. The haemofiltration device according to claim 24, **characterised in that** the first and second sensor elements are conductivity sensors.

26. The haemofiltration device according to one of claims 10 to 25, **characterised in that** the evaluation and control unit (30) is suitable for saving the blood flow Qb as the effective fluid flow which flows into the blood inlet pipe (5) and in which the change of the physical-chemical characteristic can have an effect.

27. The haemofiltration device according to claim 26, **characterised in that** the evaluation and control unit (30) is suitable for saving the dialysance D1 in a first saved set of fluids Qd1, Qb1, Qs1 and Qf1 and on the basis of these values for calculating the dialysance D2 for a second set of flows Qd2, Qb2, Qs2 and Qf2.

**28.** The haemofiltration device according to claim 27, **characterised in that** the evaluation and control unit (30) is suitable, based on several saved dialysance values Dli which were determined at various points in time i during haemofiltration treatment, and several saved dialysance values D2j which were calculated at various points in time j during haemofiltration treatment, for determining the treatment efficiency Kt achieved, by summation of the corresponding dialysance values, multiplied by the time difference to the previous or subsequent instance of measuring/calculation.

**29.** The haemofiltration device according to one of claims 10 to 28, **characterised in that** the haemofiltration device further comprises a display device on which the treatment parameter, in particular the dialysance D, interim results and/or the actual treatment efficiency Kt, can be displayed.

**Revendications**

**1.** Procédé de détermination de paramètres de traitement dans un dispositif d'hémofiltration, à l'aide d'un élément de purification de sang (1) divisé en deux cellules par une membrane semi-perméable (2), dont la première cellule (3) fait partie d'un système d'évacuation d'ultrafiltrat et dont la deuxième cellule (4) fait partie d'un circuit sanguin extracorporel, d'un système d'apport de liquide de substitution (7 ; 7a, 7b, 7c', 11, 17, 41) qui permet d'ajouter du liquide de substitution dans le circuit sanguin extracorporel, le flux de liquide Qo évacué de la première chambre (3) étant défini, une propriété physico-chimique Cs1 dans le liquide de substitution et la propriété physico-chimique correspondante Cf1 dans le liquide évacué étant définies, après quoi la propriété physico-chimique Cs est modifiée dans le liquide de substitution, la propriété physico-chimique Cs2 dans le liquide de substitution et la propriété physico-chimique Cf2 correspondante dans le liquide évacué étant de nouveau définies, après quoi sont déterminés un paramètre de traitement à partir des variables déterminées du flux de liquide évacué Qo et des propriétés physico-chimiques Cs1 et Cs2 dans le liquide de substitution et Cf1 et Cf2 dans le liquide évacué.

**2.** Procédé selon la revendication 1, **caractérisé en ce que** la modification de la propriété physico-chimique Cs se fait sous forme de degré ou de bolus.

**3.** Procédé selon la revendication 2, **caractérisé en ce que** le paramètre de traitement est la capacité dialysable de l'élément de purification du sang et est défini en fonction de l'expression suivante, où on a $\Delta Cf=Cf2-Cf1$ et $\Delta Cs=Cs2-Cs1$ :

$$D = (1 - \frac{\Delta Cf}{\Delta Cs}) \,.\, Qo.$$

**4.** Procédé selon l'une quelconque des revendications précédentes, **caractérisé en ce que** le paramètre de traitement est la concentration initiale du sang Cbi.

**5.** Procédé selon l'une quelconque des revendications précédentes, **caractérisé en ce que** le flux d'ultrafiltrat Qf extrait globalement du circuit extracorporel et le flux de liquide de substitution Qs sont définis et que le flux évacué Qo est défini à partir de la somme des deux variables.

**6.** Procédé selon l'une quelconque des revendications 1 à 4, **caractérisé en ce qu'**il est prévu en supplément une conduite d'acheminement de liquide de dialyse de flux Qd dans la première cellule (3) et que le flux évacué Qo est défini comme la somme du flux d'ultrafiltrat Qf extrait globalement du circuit extracorporel, du flux de liquide de substitution Qs et du flux acheminé Qd.

**7.** Procédé selon l'une quelconque des revendications 1 à 6, **caractérisé en ce que** le flux de sang Qb est défini en plus en tant que flux de liquide sanguin effectif qui afflue dans la conduite d'acheminement de sang et dans lequel la modification de la propriété physico-chimique peut exercer des effets.

**8.** Procédé selon la revendication 7, **caractérisé en ce que** la capacité dialysable D1 est définie pour une première série de flux Qd1, Qb1, Qs1 et Qf1 et qu'ensuite la capacité dialysable D2 est définie pour une deuxième série de flux Qd2, Qb2, Qs2 et Qf2 sur la base des valeurs de la première série de flux et de D1.

9. Procédé selon la revendication 8, **caractérisé en ce que**, sur la base de plusieurs valeurs dialysables D1i qui ont été déterminées à différents moments i pendant un traitement par hémofiltration et de plusieurs valeurs dialysables D2j qui ont été déterminées à différents moments j pendant un traitement par hémofiltration, on définit l'efficacité du traitement globalement atteinte Kt par une totalisation des valeurs dialysables respectives multipliée par la différence en temps par rapport à la mesure/au calcul précédent ou suivant.

10. Dispositif d'hémofiltration pour la réalisation du procédé selon la revendication 1,
comportant un élément de purification de sang (1) divisé en deux cellules par une membrane semi-perméable (2), dont la première cellule (3) fait partie d'un système d'évacuation d'ultrafiltrat et dont la deuxième cellule (4) fait partie d'un circuit sanguin extracorporel,
d'autres pièces du circuit sanguin extracorporel comprenant une conduite d'acheminement de sang (5) qui mène à la deuxième cellule (4) et une conduite d'évacuation de sang (6) qui évacue du sang de la deuxième cellule (4),
d'autres pièces du système d'évacuation d'ultrafiltrat comprenant une conduite d'évacuation d'ultrafiltrat (8 ; 8a, 8b, 8b') qui évacue le liquide de la première cellule (3) et un dispositif de convoyage d'ultrafiltrat (20, 45) pour l'extraction ciblée de liquide d'un flux d'évacuation Qo à travers la membrane (2) par la conduite d'évacuation d'ultrafiltrat (8 ; 8a, 8b, 8b'),
comportant un système d'apport de liquide de substitution qui comprend une conduite de liquide de substitution (7 ; 7a, 7b, 7c') qui va d'une source (11) de fourniture de liquide de substitution au circuit sanguin extracorporel et un dispositif de convoyage de substitution (41) pour le convoyage de liquide de substitution dans la conduite de liquide de substitution (7 ; 7a, 7b, 7c'),
un premier élément de détection (27) disposé sur une conduite de liquide de substitution (7 ; 7a, 7b, 7c') pour la définition d'une propriété physico-chimique Cs du liquide de substitution,
un deuxième élément de détection (28) disposé sur une conduite d'évacuation d'ultrafiltrat (8 ; 8a, 8b, 8b') pour la définition de la propriété physico-chimique correspondante Cf du liquide évacué,
**caractérisé en ce**
**qu'**il est prévu un dispositif (11) de modification de la propriété physico-chimique Cs du liquide de substitution,
**qu'**il est prévu une unité d'exploitation et de commande (30) qui enregistre d'abord les valeurs de mesure Cs1 et Cf1 des premier et deuxième éléments de détection, puis commande le dispositif (11) de modification de la propriété physico-chimique du liquide de substitution afin de provoquer une telle modification et qui enregistre de nouveau les valeurs Cs2 et Cf2 modifiées en raison de la modification des premier et deuxième éléments de détection et définit le paramètre de traitement à partir des valeurs enregistrées (Cs1, Cs2, Cf1, Cf2, Qo).

11. Dispositif d'hémofiltration selon la revendication, 10,
**caractérisé en ce que**
l'unité d'exploitation et de commande (30) commande le dispositif (11) de modification de la propriété physico-chimique du liquide de substitution de manière à ce que la modification se présente sous forme de degré ou de bolus.

12. Dispositif d'hémofiltration selon la revendication 11,
**caractérisé en ce que** le
paramètre de traitement est la capacité dialysable de l'élément de purification de sang et que l'unité d'exploitation et de commande (30) est adaptée pour définir la capacité dialysable suivant l'expression suivante, sachant que $\Delta Cf = Cf2 - Cf1$ et $\Delta Cs = Cs2 - Cs1$ :

$$D + (1 - \frac{\Delta Cf}{\Delta Cs}) \cdot Qo$$

13. Dispositif d'hémofiltration selon l'une quelconque des revendications 10 à 12,
**caractérisé en ce que** le paramètre de traitement est la concentration initiale du sang Cbi.

14. Dispositif d'hémofiltration selon l'une quelconque des revendications 10 à 13,
**caractérisé en ce que** l'unité d'exploitation et de commande (30) est adaptée pour enregistrer dans le flux de substitut Qs des valeurs de réglage/et ou de mesure pour le flux d'ultrafiltrat Qf à extraire globalement du circuit extracorporel et définir le flux d'évacuation Qo sous forme de somme de ces deux flux.

**15.** Dispositif d'hémofiltration selon l'une quelconque des revendications 10 à 13, **caractérisé en ce que** la source (11) de fourniture de liquide de substitution est aussi une source de fourniture de liquide de dialyse, d'où une conduite d'acheminement de liquide de dialyse (7 ; 7a, 7b, 7c) va vers la première cellule (3).

**16.** Dispositif d'hémofiltration selon la revendication 15, **caractérisé en ce que** l'unité d'exploitation et de commande (30) est adaptée pour enregistrer le flux d'évacuation Qo sous forme de somme des valeurs de mesure et/ou de réglage pour le flux d'ultrafiltrat Qf à extraire globalement du circuit extracorporel dans le flux de liquide de substitution Qs et dans le flux de liquide de dialyse Qd coulant dans la première cellule (3).

**17.** Dispositif d'hémofiltration selon la revendication 15 ou 16, **caractérisé en ce que** la conduite d'acheminement de liquide de substitution et la conduite d'acheminement de liquide de dialyse se présentent, dans une première zone, sous forme d'une conduite commune (7a, 7b) et, après une ramification, sous forme d'une conduite séparée (7c', 7c) dans une deuxième zone.

**18.** Dispositif d'hémofiltration selon l'une quelconque des revendications 10 à 17, **caractérisé en ce qu'**il est prévu dans la conduite de substitution (7 ; 7a, 7b, 7c') un premier dispositif d'équilibrage (17) et dans la conduite d'ultrafiltrat (8 ; 8a, 8b, 8b') un deuxième dispositif d'équilibrage (18, 45).

**19.** Dispositif d'hémofiltration selon la revendication 18, **caractérisé en ce que** la conduite d'ultrafiltrat se ramifie en une première conduite (8b) et une deuxième conduite (8b'), la première conduite (8b) s'étendant dans une première partie se présentant sous forme d'une première moitié de cellule d'équilibrage (19) du deuxième dispositif d'équilibrage, étant prévu dans la deuxième conduite (8b') un dispositif (45) de convoyage du flux d'ultrafiltrat Qf à extraire globalement du circuit extracorporel et représentant la deuxième partie du deuxième dispositif d'équilibrage et la conduite de substitution (7 ; 7a) s'étendant dans un premier dispositif d'équilibrage se présentant sous forme d'une deuxième moitié de cellule d'équilibrage (17).

**20.** Dispositif d'hémofiltration selon la revendication 18 ou 19, **caractérisé en ce que** le premier (17) et/ou le deuxième dispositif d'équilibrage (18) est conçu de manière à ce que le flux Qs+Qd soit définissable et enregistrable par l'unité d'exploitation et de commande (30).

**21.** Dispositif d'hémofiltration selon la revendication 18, 19 ou 20, **caractérisé en ce que** le premier (17) et/ou le deuxième (19, 45) dispositif d'équilibrage est conçu de manière à ce que le flux d'ultrafiltrat Qf à extraire globalement du circuit extracorporel soit définissable à partir des flux le traversant et enregistrable par l'unité d'exploitation et de commande (30).

**22.** Dispositif d'hémofiltration selon l'une quelconque des revendications 10 à 21, **caractérisé en ce que** la conduite d'acheminement de liquide substitut (7c') débouche dans la conduite d'évacuation de sang (6) (post-dilution).

**23.** Dispositif d'hémofiltration selon l'une quelconque des revendications 10 à 21, **caractérisé en ce que** la conduite d'acheminement de liquide substitut (7c') débouche dans la conduite d'acheminement de sang (5) (pré-dilution).

**24.** Dispositif d'hémofiltration selon l'une quelconque des revendications 10 à 23, **caractérisé en ce que** la propriété physico-chimique est une concentration, notamment la concentration en sodium.

**25.** Dispositif d'hémofiltration selon la revendication 24, **caractérisé en ce que** les premier et deuxième éléments de détection sont des capteurs de conductibilité.

**26.** Dispositif d'hémofiltration selon l'une quelconque des revendications 10 à 25, **caractérisé en ce que** l'unité d'exploitation et de commande (30) est adaptée pour enregistrer le flux de sang Qb en tant que flux de liquide effectif dans lequel afflue la conduite d'acheminement de sang (5) et dans lequel la modification des propriétés physico-chimiques peut avoir un effet.

27. Dispositif d'hémofiltration selon la revendication 26, **caractérisé en ce que** l'unité d'exploitation et de commande (30) est adaptée pour définir et enregistrer la capacité dialysable D1 dans une première série enregistrée de flux Qd1, Qb1, Qs1 et Qf1 et pour calculer sur la base de ces valeurs la capacité dialysable D2 pour une deuxième série de flux Qd2, Qb2, Qs2 et Qf2.

28. Dispositif d'hémofiltration selon la revendication 27, **caractérisé en ce que** l'unité d'exploitation et de commande (30) est adaptée, sur la base de plusieurs valeurs dialysables enregistrées Dli qui ont été déterminées à différents moments i pendant un traitement par hémofiltration et de plusieurs valeurs dialysables enregistrées D2j qui ont été calculées pour différents moments j pendant un traitement par hémofiltration, pour définir l'efficacité de traitement Kt globalement atteinte par totalisation des valeurs dialysables respectives multipliées à la différence en temps par rapport à la mesure/au calcul précédent ou suivant.

29. Dispositif d'hémofiltration selon l'une quelconque des revendications 10 à 28, **caractérisé en ce que** le dispositif d'hémofiltration comprend de plus un dispositif d'affichage sur lequel peuvent s'afficher le paramètre de traitement, notamment la capacité dialysable D, les résultats intermédiaires et/ou l'efficacité de traitement atteinte Kt.

**Fig. 1**

Qs(+Qd), Cs

7

Qb-Qf, Cbo

6

4

1

2

3

50

8

Qf+Qs(+Qd), Cf

5

Qb, Cbi

**Fig. 2a**

Qs(+Qd), Cs

7

Qb-Qf, Cbo

6

4

1

2

3

50

8

Qf+Qs(+Qd), Cf

5

Qb, Cbi

**Fig. 2b**

Fig. 3